(19) Europäisches Patentamt
European Patent Office
Office européen des brevets

(11) EP 1 773 770 B1

# (12) EUROPEAN PATENT SPECIFICATION

(45) Date of publication and mention of the grant of the patent:
**01.04.2009 Bulletin 2009/14**

(21) Application number: **05764412.2**

(22) Date of filing: **21.07.2005**

(51) Int Cl.:
*C07D 209/42* (2006.01) *C07D 401/12* (2006.01)
*C07D 405/12* (2006.01) *A61K 31/405* (2006.01)
*A61P 25/00* (2006.01)

(86) International application number:
**PCT/HU2005/000078**

(87) International publication number:
**WO 2006/010965 (02.02.2006 Gazette 2006/05)**

## (54) INDOLE-2-CARBOXAMIDINE DERIVATIVES AS NMDA RECEPTOR ANTAGONISTS

INDOL-2-CARBOXAMIDIN-DERIVATE ALS ANTAGONISTEN DER NMDA-REZEPTOREN

DERIVES DE INDOLE-2 -CARBOXAMIDINE UTILISES COMME ANTAGONISTES DU RECEPTEUR NMDA

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **29.07.2004 HU 0401523**

(43) Date of publication of application:
**18.04.2007 Bulletin 2007/16**

(73) Proprietor: **Richter Gedeon Nyrt.**
**1103 Budapest (HU)**

(72) Inventors:
- **BORZA, István**
**H-1186 Budapest (HU)**
- **HORVÁTH, Csilla**
**H-1104 Budapest (HU)**
- **FARKAS, Sándor**
**H-1103 Budapest (HU)**
- **NAGY, József**
**H-1138 Budapest (HU)**
- **KOLOK, Sándor**
**H-1195 Budapest (HU)**

(74) Representative: **HOFFMANN EITLE**
**Patent- und Rechtsanwälte**
**Arabellastraße 4**
**81925 München (DE)**

(56) References cited:
**WO-A-00/67751**
**WO-A-00/67755**
**WO-A-01/98262**
**WO-A-02/34718**
**US-A1- 2003 130 354**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to new indole-2-carboxamidine derivatives which are antagonists of NMDA receptor or are intermediates for preparing thereof.

## Background of the invention.

N-methyl-D-aspartate (NMDA) receptors are ligand-gated cation-channels widely expressed in the central nervous system. NMDA receptors are involved in developmental and plastic changes of the central nervous system. Overactivation of NMDA receptors by glutamate, their natural ligand, can lead to calcium overload of cells. This triggers a cascade of intracellular events that alters the cell function and ultimately may lead to death of neurones [TINS, 10, 299-302 (1987)]. Antagonists of the NMDA receptors may be used for treating many disorders that are accompanied with excess release of glutamate.

The NMDA receptors are heteromeric assemblies built up from at least one NR1 subunit together with one or more of the four NR2 subunits (NR2A-D). Recently, NR3A and NR3B have been reported. Both spatial distributions in the CNS and the pharmacological sensitivity of NMDA receptors built up from various NR2 subunits are different. Particularly interesting of these is the NR2B subunit due to its restricted distribution (highest densities in the forebrain and substantia gelatinosa of the spinal cord). Compounds selective for this subtype are available and have been proved to be effective in animal models of stroke [Stroke, 28, 2244-2251 (1997)], traumatic brain injury [Brain Res., 792, 291-298 (1998)], Parkinson's disease [Exp. Neurol.,163, 239-243 (2000)], neuropathic and inflammatory pain [Neuropharmacology, 38, 611-623 (1999)]. Moreover, NR2B subtype selective antagonists of NMDA receptors are expected to possess more favorable side effect profile than non-selective antagonists of NMDA receptors. The NR2B selective compound CP-101,606 when examined in a clinical trial was well tolerated and did not have the undesirable effects characteristic of non-selective antagonists, namely psychotomimetic effects, hallucinations, dysphoria at a dose that reduced neuropathic pain (C.N. Sang, et al. Program No. 814.9. 2003 Abstract Viewer/Itinerary Planner. Washington, DC: Society for Neuroscience, 2003. Online).

NR2B subtype selective NMDA antagonism can be achieved with compounds that specifically bind to, and act on, an allosteric modulatory site of the NR2B subunit containing receptors. This binding site can be characterised by displacement (binding) studies with specific radioligands, such as [$^3$H]-Ro 25,6981 [J. Neurochem., 70, 2147-2155 (1998)].

Close structure analogs of the carboxylic acid amidine derivatives of formula (I) are unknown from the literature. Although, some different carboxamidine derivatives are described as NR2B subtype selective NMDA antagonists in the following publications:

Cinnamamidines are described in patent No. WO 200198262 and Bioorg. Med. Chem. Letters, 13, 693-696. (2003).
Benzamidines are described in patent No. WO 200067751 and Bioorg. Med. Chem. Letters, 13, 693-696. (2003) as well as Bioorg. Med. Chem. Letters, 13, 697-700. (2003).

## Summary of the invention

Surprisingly it was found that the new indole-2-carboxamidine derivatives of formula (I) of the present invention are functional antagonists of NMDA receptors of rat cortical neurons with IC50 below 1 micromole. This effect is due to their inhibition of NR2B containing receptors, since they are ineffective on NR2A subunit containing NMDA receptors at 10 micromole. Therefore, they are believed to be NR2B subtype specific NMDA antagonists, and possess therapeutic utility.

## Detailed description of the invention

The present invention relates therefore first to new indole-2-carboxamidine derivatives of formula (I)

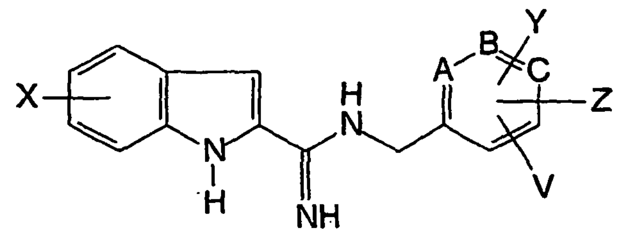

(I)

- wherein the meaning of

| | |
|---|---|
| X | is hydrogen or halogen atom, $C_1$-$C_4$ alkyl, $C_1$-$C_4$ alkoxy, trifluoromethyl group, |
| Y, V and Z | independently are hydrogen or halogen atom, hydroxy, cyano, $C_1$-$C_4$. alkylsulfonamido optionally substituted by a halogen atom or halogen atoms, $C_1$-$C_4$ alkanoylamido optionally substituted by a halogen atom or halogen atoms, trifluoromethyl, trifluoromethoxy, $C_1$-$C_4$ alkyl, $C_1$-$C_4$ alkoxy group, or |
| the neighboring V and Z | groups in given case together with one or more identical or different additional hetero atom and -CH= and/or -$CH_2$- groups can form an optionally substituted 4-7 membered homo- or heterocyclic ring, preferably benzene, dioxolane ring, |

A, B and C independently are substituted carbon atom or one of them is nitrogen atom, and the salts thereof.

Further objects of the invention are the processes for producing indole-2-carboxamidine derivatives of formula (I), and the pharmaceutical manufacture of medicaments containing these compounds, as well as the process of treatments with these compounds, which means administering to a mammal to be treated - including human - effective amount/amounts of indole-2-carboxamidine derivatives of formula (I) of the present invention as such or as medicament.

The new indole-2-carboxamidine derivatives of formula (I) of the present invention are highly effective and selective antagonists of NMDA receptor, and moreover most of the compounds are selective antagonist of NR2B subtype of NMDA receptor.

According to the invention the indole-2-carboxamidine derivatives of formula (I) are synthesized by reacting a carboximidic acid alkyl ester salt of formula (II)

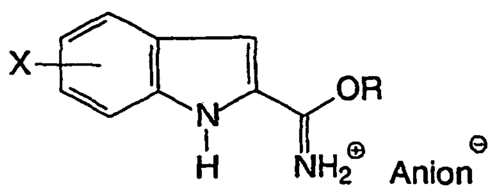

(II)

- wherein the meaning of X is as given in claim 1, Anion means one equivalent of an anion, R is $C_1$-$C_4$ alkyl- with an aralkylamine of formula (III)

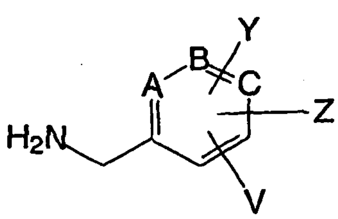

(III)

- wherein the meaning of A, B, C, Y, V, Z are as given before for the formula (I), then the so obtained indole-2-carboxamidine derivatives of formula (I)- wherein the meaning of A, B, C, Y, V, Z are as defined for the formula (I) - in given case are transformed into another compounds of formula (I) by introducing new substituents and/or

modifying or removing the existing ones, and/or by forming salt and/or by liberating the compound from salts by known methods.

The compounds of this invention are readily prepared by reacting the appropriate carboximidic acid alkyl ester hydrochloride with an appropriate aralkylamine in a reaction-insert solvent at a suitable temperature, in the presence of a base. Representative solvents for these reactions are ethanol, methanol, methylene chloride, ethylene dichloride, tetrahydrofuran.

The desired carboximidic acid alkyl ester hydrochlorides (e.g. ethyl ester) are conveniently prepared by Pinner reaction i.e. by reacting the corresponding nitril with saturated hydrochloric acid solution of alcohol. The nitril reactants needed for the preparation of the carboximidic acid alkyl ester hydrochloride reactants are prepared by reaction of the corresponding amides with phosphorus oxychloride. The acid amides are prepared by amidation of the corresponding acid chlorides according to well known procedures. The acid chlorides are prepared by reaction of the appropriate carboxylic acid with thionyl chloride, the latter generally serving as reactant and solvent as well. In the reaction of compound of formula (II) with compound of formula (III) the applied base is the molar excess of the reagent aralkylamine of formula (III). The necessary reaction time is 1-24 h, preferably 6 h. The preferred -reaction temperature is from about 20 °C to about 30 °C. The reaction mixture is purified by column chromatography using Kieselgel 60 (Merck) as adsorbent and a proper eluent. The proper fractions are acidified with hydrochloric acid and concentrated to give the hydrochloric acid salt of the pure product. The quality and the quantity of the product are determined by HPLC-MS method.

The 1H-indole-2-carboxylic acids needed for the preparation of the carboximidic acid alkyl ester hydrochloride reactants of formula (II) and the benzil amines of formula (III) are either commercially available.

## Experimental protocols

### expression of recombinant NMDA receptors

To prove NR2B selectivity of our compounds, we tested them on cell lines stably expressing recombinant NMDA receptors with subunit compositions of NR1(-3)/NR2A. cDNAs of human NR1(-3) and NR2A subunits subcloned into inducible mammalian expression vectors were introduced into HEK 293 cells lacking NMDA receptors using a cationic lipid-mediated transfection method [Biotechniques, 1997 May; 22(5): 982-7; Neurochemistry International, 43, 19-29. (2003)]. Resistance to neomycin and hygromycin was used to screen for clones possessing both vectors and monoclonal cell lines were established from the clones producing the highest response to NMDA exposure. Compounds were tested for their inhibitory action on NMDA evoked cytosolic calcium elevations in fluorescent calcium measurements. Studies were performed 48-72 h after addition of the inducing agent. Ketamine (500 μM) was also present during the induction in order to prevent cytotoxicity.

### Assessment of NMDA antagonist potency *in vitro* by measurement of intracellular calcium concentration with a plate reader fluorimeter in rat cortical cell culture

The intracellular calcium measurements were carried out on primary neocortical cell cultures derived from 17 day old Charles River rat embryos (for the details on the preparation of neocortical cell culture see Johnson, M.I.; Bunge, R.P. (1992): Primary cell cultures of peripheral and central neurons and glia. In: Protocols for Neural Cell Culture, eds: Fedoroff, S.; Richardson A., The Humana Press Inc., 51-75.) After isolation the cells were plated onto standard 96-well microplates and the cultures were maintained in an atmosphere of 95% air-5% $CO_2$ at 37°C until the calcium measurements.

The cultures were used for the intracellular calcium measurements after 3-7 days in vitro. Before the measurement the cells were loaded with a fluorescent $Ca^{2+}$-sensitive dye, Fluo-4/AM (2μM). To stop the loading the cells were washed twice with the solution used for the measurement (140 mM NaCl, 5 mM KCl, 2 mM $CaCl_2$, 5 mM HEPES, 5 mM HEPES-Na, 20 mM glucose, 10 μM glycine, pH=7.4). After washing the test compounds were added to the cells in the above solution (90 μl/well). Intracellular calcium measurements were carried out with a plate reader fluorimeter: elevation of Fluo-4-fluorescence and so, intracellular calcium was induced by application of 40 μM NMDA. Inhibitory potency of the test compounds was assessed by measuring the reduction in the calcium elevation in the presence of different concentrations of the compounds.

Dose-response curves and $IC_{50}$ values were calculated by using data derived from at least three independent experiments. Inhibitory potency of a compound at a single concentration point was expressed as percent inhibition of the NMDA response. Sigmoidal concentration-inhibition curves were fit to the data and $IC_{50}$ values were determined as the concentration that produces half of the maximal inhibition caused by the compound.

In Table 1, NR2B antagonist potencies of the most effective compounds of this invention determined in this test are listed. The results with the selective NR2B antagonist CI-1041 and the non-selective NMDA receptor antagonist

MK-801, used as reference compounds, are given in Table 2.

**Table 1**

| NMDA antagonist activity of compounds measured by fluorimetric method on cortical cells or NR1-3/NR2A subunit expressing cells | | | | |
|---|---|---|---|---|
| Compound of Table 3 | Cortical cells | | NR1-3/NR2A | |
| | $IC_{50}$ [nM] | N | % inhibition at 15 μM | n |
| 36 | 5.4 | 3 | 32.1 | **1** |
| 147 | 26 | 5 | 21.7 | **1** |
| 134 | 100 | 4 | 6.1 | **1** |
| 19 | 75 | 5 | | |
| 57 | 211 | 3 | | |
| 59 | 51 | 3 | | |
| 81 | 432 | 4 | | |
| 83 | 369 | 4 | | |
| 87 | 422 | 6 | | |
| 92 | 319 | 4 | | |
| 94 | 213 | 4 | | |
| 97 | 125 | 4 | | |
| 118 | 224 | 5 | | |
| 119 | 401 | 3 | | |
| 123 | 129 | 4 | | |
| 127 | 373 | 6 | | |
| 128 | 220 | 3 | | |
| 129 | 52 | 4 | | |
| 130 | 266 | 5 | | |
| 131 | 57 | 4 | | |
| 25 | 277 | 5 | | |
| 26 | 418 | 6 | | |
| 29 | 232 | 3 | | |
| 30 | 224 | 3 | | |
| 31 | 143 | 4 | | |
| 32 | 170 | 4 | | |
| 35 | 341 | 3 | | |
| 67 | 359 | 3 | | |
| 68 | 160 | 4 | | |
| 70 | 594 | 5 | | |
| 71 | 453 | 3 | | |
| 72 | 17 | 3 | | |

(continued)

| NMDA antagonist activity of compounds measured by fluorimetric method on cortical cells or NR1-3/NR2A subunit expressing cells | | | | |
|---|---|---|---|---|
| Compound of Table 3 | Cortical cells | | NR1-3/NR2A | |
| | $IC_{50}$ [nM] | N | % inhibition at 15 μM | n |
| 103 | 257 | 4 | | |
| 107 | 493 | 3 | | |
| 126 | 519 | 5 | | |
| 109 | 218 | 3 | | |
| 110 | 702 | 3 | | |
| 111 | 417 | 3 | | |
| 112 | 69 | 4 | | |
| 138 | 232. | 3 | | |
| 139 | 397 | 4 | | |
| 142 | 202 | 3 | | |
| 143 | 158 | 3 | | |
| 145 | 318 | 3 | | |
| 146 | 287 | 4 | | |
| 108 | 742 | 4 | | |
| 106 | 198 | 3 | | |
| 38 | 159 | 3 | | |
| 73 | 209 | 5 | | |

**Table 2**

| NMDA antagonist activity of reference compounds measured by fluorimetric method on cortical cells or NR1-3/NR2A subunit expressing cells | | | | |
|---|---|---|---|---|
| | Cortical cells | | NR1-3/NR2A | |
| Code of reference compounds | $IC_{50}$ [nM] | n | % inhibition at 10 μM | n |
| CI-1041 | 6.6 | 4 | 21.0 | 1 |
| Co-101244 | 23 | 3 | -8.7 | 1 |
| EMD 95885 | 35 | 1 | 0.1 | 1 |
| CP 101,606 | 41 | 3 | 2.5 | 1 |
| Ro 25.6981 | 159 | 4 | 1.0 | 1 |
| Erythro-ifenprodil | 483 | 5 | -2.7 | 1 |

(continued)

| NMDA antagonist activity of reference compounds measured by fluorimetric method on cortical cells or NR1-3/NR2A subunit expressing cells | | | | |
|---|---|---|---|---|
| | **Cortical cells** | | **NR1-3/NR2A** | |
| **Code of reference compounds** | **$IC_{50}$ [nM]** | **n** | **% inhibition at 10 μM** | **n** |
| MK-801 | 37 | 3 | $IC_{50}$=386 nM | 2 |

The reference compounds are as follows:
CI-1041: 6-{2-[4-(4-fluoro-benzyl)-piperidin-1-yl]-ethanesulfinyl}-3*H*-benzooxazol-2-one
Co 101244: 1-[2-(4-hydroxyphenoxy)ethyl]-4-hydroxy-4-(4-methylbenzyl)piperidine
EMD 95885: 6-[3-(4-fluorobenzyl)piperidine-1-yl]propionyl]-2,3-dihydro-benzoxazol-2-on
CP-101;606: (1S,2S)-1-(4-hydroxyphenyl)-2-(4-hydroxy-4-phenylpiperidine-1-yl)-1-propanol
Co-111103: 1-(2-(4-hydroxyphenoxy)ethyl]-4-(4-fluorobenzyl)piperidine
Ro 256981: R-(R*,S*)-1-(4-hydroxyphenyl)-2-methyl-3-[4-(phenylmethyl)piperidin-1-yl]-1-propanol.
Ifenprodil: *erythro*-2-(4-benzylpiperidino)-1-(4-hydroxyphenyl)-1-propanol
MK-801: (+)-5-Methyl-10,11-dihydro-5H-dibenzo[a,d]cyclohepten-5,10-imine

Disorders which may be beneficially treated with NMDA antagonists acting at NR2B site, as reviewed recently by Loftis [Pharmacology & Therapeutics, **97**, 55-85 (2003)] include schizophrenia, Parkinson's disease, Huntington's disease, excitotoxicity evoked by hypoxia and ischemic, seizure disorders, drug abuse, and pain, especially neuropathic, inflammatory and visceral pain of any origin [Eur. J. Pharmacol., **429**,71-78 (2001)].

Due to their reduced side effect liability compared to non-selective NMDA antagonists, NR2B selective antagonists may have utility in diseases where NMDA antagonist may be effective, such as amyotrophic lateral sclerosis [Neurol. Res., 21, 309-12 (1999)], withdrawal syndromes of e.g. alcohol, opioids or cocaine [Drug and Alcohol Depend., 59; 1-15 (2000)], muscular spasm [Neurosci. Lett., 73, 143-148 (1987)], dementia of various origins [Expert Opin. Investig. Drugs, 9, 1397-406 (2000)], anxiety, depression, migraine, hypoglycemia, degenerative disorders of the retina (e.g. CMV retinitis), glaucoma, asthma, tinnitus, hearing loss [Drug News Perspect 11, 523-569 (1998) and WO 00/00197 international patent application].

Accordingly, effective amounts of the compounds of the invention may be beneficially used for the treatment of traumatic injury of brain or spinal cord, tolerance and/or dependence to opioid treatment of pain, development of tolerance, decrease of abuse potential and withdrawal syndromes of drugs of abuse e.g. alcohol, opioids or cocaine, ischemic CNS disorders, chronic neurodegenerative disorders, such as e.g. Alzheimer's disease, Parkinson's disease, Huntington's disease, pain and chronic pain states, such as e.g. neuropathic pain.

The compounds of the invention as well as their pharmaceutically acceptable salts can be used as such or suitably in the form of pharmaceutical compositions. These compositions (drugs) can be in solid, liquid or semiliquid form and pharmaceutical adjuvant and auxiliary materials can be added, which are commonly used in practice, such as carriers, excipients, diluents, stabilizers, wetting or emulsifying agents, pH- and osmotic pressure-influencing, flavoring or aromatizing, as well as formulation-promoting or formulation-providing additives.

The dosage required to exert the therapeutical effect can vary within wide limits and will be fitted to the individual requirements in each of the particular cases, depending on the stage of the disease, the condition and the bodyweight of the patient to be treated, as well as the sensitivity of the patient against the active ingredient, route of administration and number of daily treatments. The actual dose of the active ingredient to be used can safely be determined by the attending physician skilled in the art in the knowledge of the patient to be treated.

The pharmaceutical compositions containing the active ingredient according to the present invention usually contain 0.01 to 100 mg of active ingredient in a single dosage unit. It is, of course possible that the amount of the active ingredient in some compositions exceeds the upper or lower limits defined above.

The solid forms of the pharmaceutical compositions can be for example tablets, dragées, capsules, pills or lyophilized powder ampoules useful for the preparation of injections. Liquid compositions are the injectable and infusable compositions, fluid medicines, packing fluids and drops. Semiliquid compositions can be ointments, balsams, creams, shaking mixtures and suppositories.

For the sake of a simple administration it is suitable if the pharmaceutical compositions comprise dosage units containing the amount of the active ingredient to be administered once, or a few multiples or a half, third or fourth part thereof. Such dosage units are e.g. tablets, which can be powdered with grooves promoting the halving or quartering of the tablet in order to exactly administer the required amount of the active ingredient.

Tablets can be coated with an acid-soluble layer in order to assure the release of the active ingredient content

after leaving the stomach. Such tablets are enteric-coated. A similar effect can be achieved also by encapsulating the active ingredient.

The pharmaceutical compositions for oral administration can contain e.g. lactose or starch as excipients, sodium carboxymethylcellulose, methylcellulose, polyvinyl pyrrolidone or starch paste as binders or granulating agents. Potato starch or microcrystalline cellulose is added as disintegration agents, but ultraamylopectin or formaldehyde casein can also be used. Talcum, colloidic silicic acid, stearin, calcium or magnesium stearate can be used as antiadhesive and lubricants.

The tablet can be manufactured for example by wet granulation, followed by pressing. The mixed active ingredients and excipients, as well as in given case part of the disintegrants are granulated with an aqueous, alcoholic or aqueous alcoholic solution of the binders in an appropriate equipment, then the granulate is dried. The other disintegrants, lubricants and antiadhesive agents are added to the dried granulate, and the mixture is pressed to a tablet. In given case the tablets are made with halving groove to ease the administration.

The tablets can be made directly from the mixture of the active ingredient and the prosper auxiliaries by pressing. In given case, the tablets can be coated by using additives commonly used in the pharmaceutical practice, for example stabilizers, flavoring, coloring agents, such as sugar, cellulose derivatives (methyl- or ethylcellulose, sodium carboxymethylcellulose, etc), polyvinyl pyrrolidone, calcium phosphate, calcium carbonate, food coloring agents, food laces, aroma agents, iron oxide pigments, etc. In the case of capsules the mixture of the active ingredient and the auxiliaries is filled into capsules.

Liquid oral compositions, for example suspensions, syrups, elixirs can be made by using water, glycols, oils, alcohols, coloring and flavoring agents.

For rectal administration the composition is formulated in suppositories or clysters. The suppository can contain beside the active ingredient a carrier, so called adeps pro suppository. Carriers can be vegetable oils, such as hydrogenated vegetable oils, triglycerides of $C_{12}$-$C_{18}$ fatty acids (preferably the carriers under the trade name Witepsol). The active ingredient is homogeneously mixed with the melted adeps pro suppository and the suppositories are moulded.

For parenteral administration the composition is formulated as injection solution. For manufacturing the injection solution the active ingredients are dissolved in distilled water and/or in different organic solvents, such as glycolethers, in given case in the presence of solubilizers, for example polioxyethylensorbitane-monolaurate, -monooleate, or monostearate (Tween 20, Tween 60, Tween 80). The injection solution can also contain different auxiliaries, such as conserving agents, for example ethylendiamine tetraacetate, as well as pH adjusting agents and buffers and in given case local anaesthetic, e.g. lidocain. The injection solution containing the active ingredient of the invention is filtered before it is filled into ampoules, and it is sterilized after filling.

If the active ingredient is hygroscopic, then it can be stabilized by liophylization.

The following examples illustrate the invention without the intention of limitation anyway.

## Characterization method

Compounds of the present invention were characterized by high performance liquid chromatography coupled to mass selective detector (LC/MS) using HP 1100 Binary Gradient chromatography system with Microplate Sampler (Agilent, Waldbronn), controlled by ChemStation software. HP diode array detector was used to acquire UV spectra at 225 and 240 nm. All experiments were performed using HP MSD (Agilent, Waldbronn) single quadruple spectrometer equipped with an electrospray ionization source to determine the structure.

The synthesized products were dissolved in 1 ml of DMSO (Aldrich, Germany). 100 μl of each solution was diluted with DMSO to 1000 μl volume. Analytical chromatographic experiments were performed on Discovery RP C-16 Amide, 5 cm X 4.6 mm X 5 μm column from Supelco (Bellefonte, Pennsylvania) with a flow rate of 1 ml/minute for qualification. The obtained compounds were characterized by their k' value (purity, capacity factor). k' factors are evaluated by the following formula:

$$k' = (t_R - t_0) / t_0 .$$

where k'= capacity factor, $t_R$ = retention time and to = eluent retention time.

The A eluent was trifluoroacetic acid (TFA) (Sigma, Germany) containing 0.1% water, the B eluent was 95% acetonitrile (Merck, Germany) containing 0.1% TFA and 5% A eluent. Gradient elution was used, starting with 100% A eluent and processing to 100% B eluent over a period of 5 minutes.

The process according to our invention is illustrated in detail by the following not limiting examples.

## Example 1

**N-Benzyl-1H-indole-2-carboxamidine hydrochloride**

1a) 1H-Indole-2-carboxylic acid amide

A mixture of 10.0 g (62 mmol) of 1H-indole-2-carboxylic acid (Aldrich) , 10.0 ml (76 mmol) of thionyl chloride, 100 ml of chloroform and 1 drop of dimethylformamide is refluxed for 2h. The reaction mixture is cooled to 20 ˚C, poured into a mixture of 100 ml of 25 % ammonia solution and 100 g of ice, then stirred for 2h. The precipitated product is filtered off and washed with water to yield 8.96 g (90 %) of the title compound. Mp.: 231-232 ˚C.

1b) 1H-Indole-2-carbonitrile

A mixture of 8.96 g (56 mmol) of 1H-indole-2-carboxylic acid amide, 26.0 ml (279 mmol) of phosphorus oxychloride and 230 ml of chloroform is refluxed for 2h. The reaction mixture is cooled to 20 ˚C, poured into 100 ml of water and stirred for 1h. After separation the organic layer is dried over sodium sulfate and concentrated. The residue is purified by column chromatography using Kieselgel 60 (Merck) as adsorbent and hexane-ethyl acetate = 4: 1 as eluent to yield 5.28 g (66.4 %) of the title compound. Mp.: 94-95˚C.

1c) 1H-Indole-2-carboximidic acid methyl ester hydrochloride

To a solution of 20 ml of saturated hydrochloric acid in ethanol 2.23 g (15.7 mmol) of 1H-indole-2-carbonitrile is added. The reaction mixture is stirred at 20 ˚C for 2 h, then concentrated and the residue is crystallized with ether to yield 3.1 g (82 %) of the title compound. Mp.: 170 ˚C .

1d) N-Benzyl-1H-indole-2-carboxamidine hydrochloride

To a solution of 32.1 mg (0.3 mmol) of benzylamine in 1 ml of ethanol, 28.2 mg (0.15 mmol) of 1H-indole-2-carboximidic acid ethyl ester hydrochloride in 1 ml of ethanol is added. The reaction mixture is stirred at room temperature for 6 h, and then concentrated. The residue is purified by column chromatography using Kieselgel 60 (Merck) as adsorbent and toluene : methanol = 4 : 1 as eluent. The fractions containing the product are acidified with hydrochloric acid and concentrated. The quality and the quantity of the product are determined by HPLC-MS method as described above . k' = 2.46.

The substituted 1H-indole-2-carboximidic acid ethyl ester hydrochloride derivatives covered in the Table 3, which are representatives of the compounds of formula (I) are prepared from the corresponding 1H-indole-2-carboxylic acid according to the method describe in Example 1 and are characterized using the above characterizing method.

**Table 3**

| 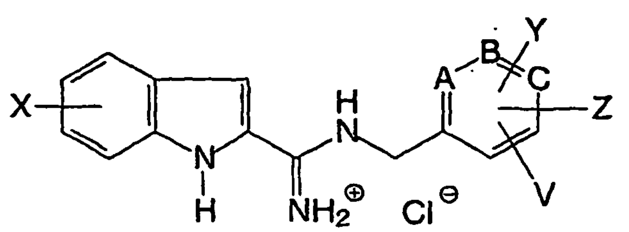 (I) | | | |
|---|---|---|---|
| **No.** | 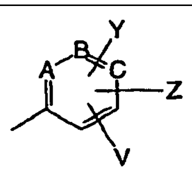 | **X** | **k'** |
| 1 | 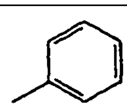 | H | 2.46 |

(continued)

| No. | 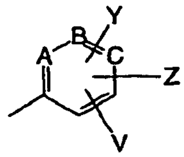 | X | k' |
|---|---|---|---|
| 2 | 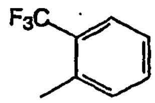 | H | 2.78 |
| 3 | 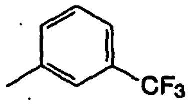 | H | 3.01 |
| 4 | 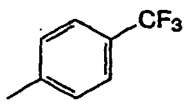 | H | 3.02 |
| 5 | 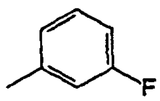 | H | 2.51 |
| 6 | 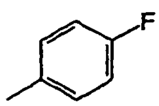 | H | 2.56 |
| 7 | 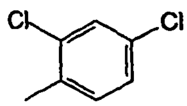 | H | 3.01 |
| 8 | 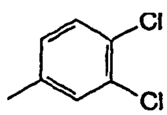 | H | 3.14 |
| 9 | 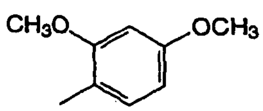 | H | 2.82 |
| 10 | 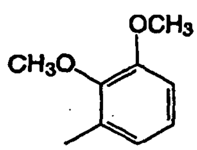 | H | 2.66 |
| 11 | 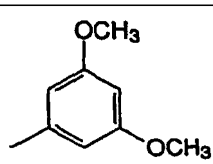 | H | 2.69 |
| 12 | 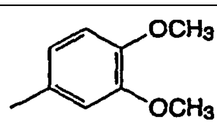 | H | 2.47 |
| 13 | 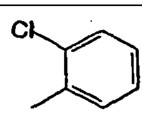 | H | 2.67 |

(continued)

| No. | 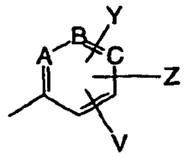 | X | k' |
|---|---|---|---|
| 14 | 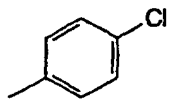 | H | 2.76 |
| 15 | 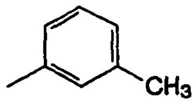 | H | 2.73 |
| 16 | 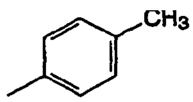 | H | 2.8 |
| 17 | 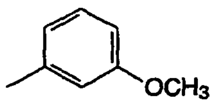 | H | 2.61 |
| 18 | 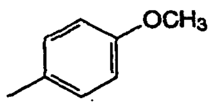 | H | 2.58 |
| 19 | 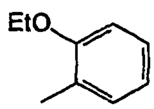 | H | 2.85 |
| 20 | 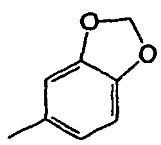 | H | 2.56 |
| 21 | 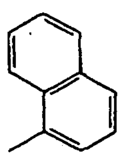 | H | 4.01 |
| 22 | 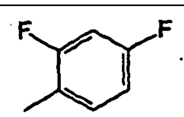 | H | 3.57 |
| 23 | 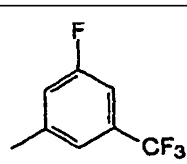 | H | 3.98 |
| 24 | 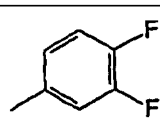 | H | 3.61 |

(continued)

| No. | 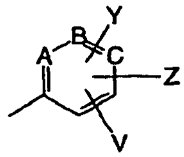 | X | k' |
|---|---|---|---|
| 25 | 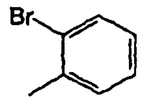 | H | 3.86 |
| 26 | 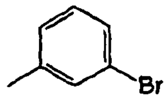 | H | 3.83 |
| 27 | 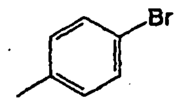 | H | 3.88 |
| 28 | 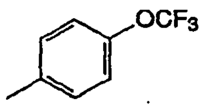 | H | 3.99 |
| 29 | 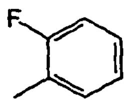 | H | 3.54 |
| 30 | 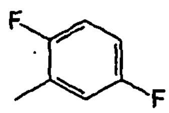 | H | 3.53 |
| 31 | 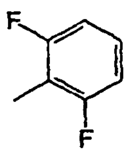 | H | 3.52 |
| 32 | 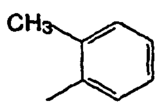 | H | 3.73 |
| 33 | 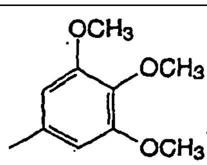 | H | 3.35 |
| 34 | 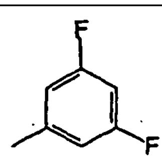 | H | 3.64 |
| 35 | 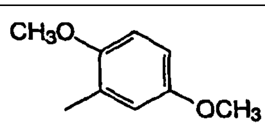 | H | 3.66 |

(continued)

| No. | 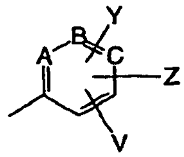 | X | k' |
|---|---|---|---|
| 36 | 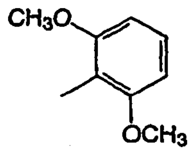 | H | 3.67 |
| 37 | 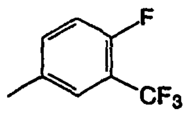 | H | 3.671 |
| 38 | 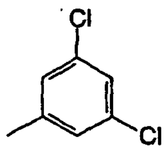 | H | 3.913 |
| 39 | 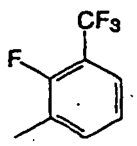 | H | 3.94 |
| 40 | 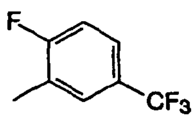 | H | 3.67 |
| 41 | 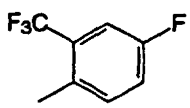 | H | 3.45 |
| 42 | 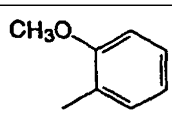 | H | 5.23 |
| 43 | 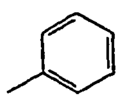 | 5-F | 2.53 |
| 44 | 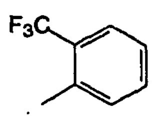 | 5-F | 2.91. |
| 45 | 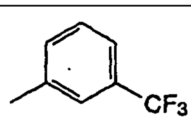 | 5-F | 3.09 |
| 46 | 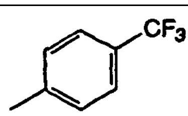 | 5-F | 3.06 |

(continued)

| No. | 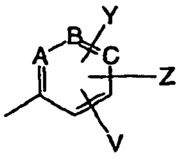 | X | k' |
|---|---|---|---|
| 47 | 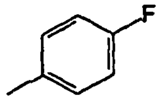 | 5-F | 2.64 |
| 48 | 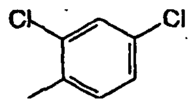 | 5-F | 3.13 |
| 49 | 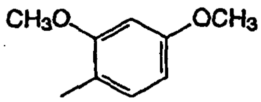 | 5-F | 2.91 |
| 50 | 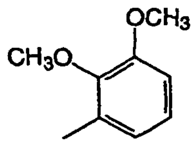 | 5-F | 2.77 |
| 51 | 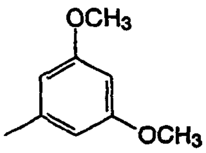 | 5-F | 2.81 |
| 52 | 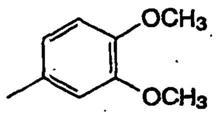 | 5-F | 2.61 |
| 53 | 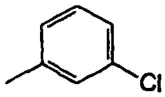 | 5-F | 2.91 |
| 54 | 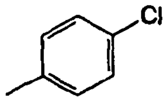 | 5-F | 2.77 |
| 55 | 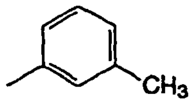 | 5-F | 2.81 |
| 56 | 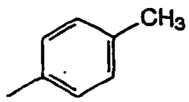 | 5-F | 2.61 |
| 57 | 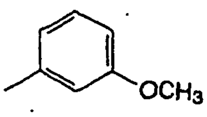 | 5-F | 2.91 |
| 58 | 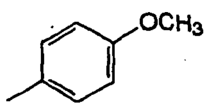 | 5-F | 2.77 |

(continued)

| No. | 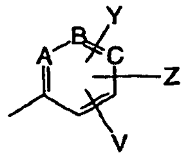 | X | k' |
|---|---|---|---|
| 59 | 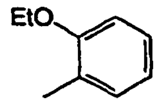 | 5-F | 2.81 |
| 60 | 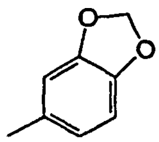 | 5-F | 2.61 |
| 61 | 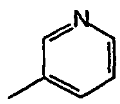 | 5-F | 0.75 |
| 62 | 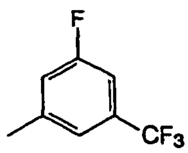 | 5-F | 3.98 |
| 63 | 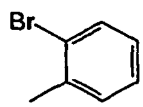 | 5-F | 3.87 |
| 64 | 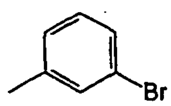 | 5-F | 3.9 |
| 65 | 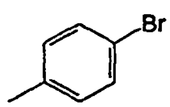 | 5-F | 3.83 |
| 66 | 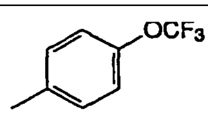 | 5-F | 4.05 |
| 67 | 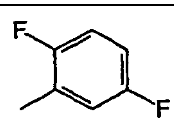 | 5-F | 3.66 |
| 68 | 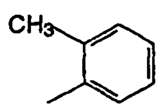 | 5-F | 3.7 |
| 69 | 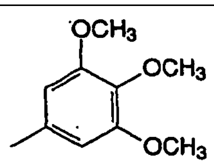 | 5-F | 3.16 |

(continued)

| No. | 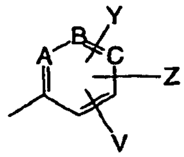 | X | k' |
|---|---|---|---|
| 70 | 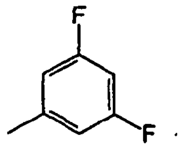 | 5-F | 3.75 |
| 71 | 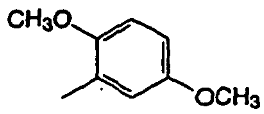 | 5-F | 3.7 |
| 72 | 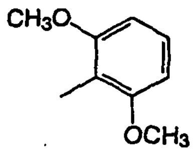 | 5-F | 3.8 |
| 73 | 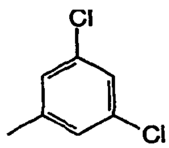 | 5-F | 3.947 |
| 74 | 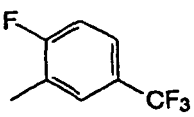 | 5-F | 3.768 |
| 75 | 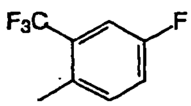 | 5-F | 3.689 |
| 76 | 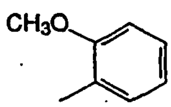 | 5-F | 5.63 |
| 77 | 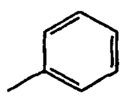 | 5-Cl | 2.79 |
| 78 | 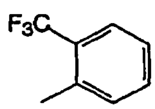 | 5-Cl | 3.08 |
| 79 | 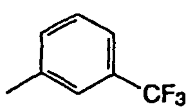 | 5-Cl | 3.29 |
| 80 | 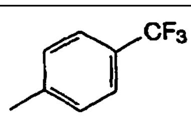 | 5-Cl | 3.26 |

(continued)

| No. | 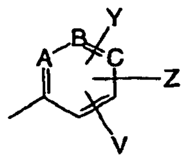 | X | k' |
|---|---|---|---|
| 81 | 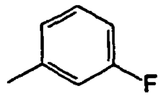 | 5-Cl | 2.91 |
| 82 | 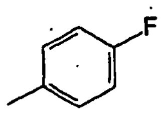 | 5-Cl | 2.91 |
| 83 | 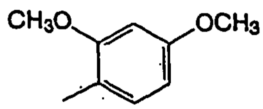 | 5-Cl | 3.02 |
| 84 | 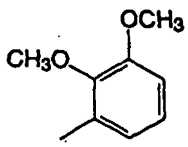 | 5-Cl | 2.56 |
| 85 | 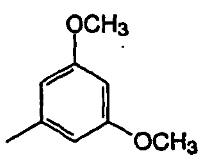 | 5-Cl | 3.07 |
| 86 | 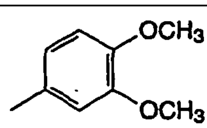 | 5-Cl | 2.86 |
| 87 | 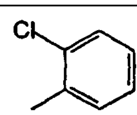 | 5-Cl | 3.03 |
| 88 | 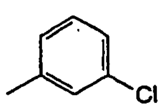 | 5-Cl | 3.1 |
| 89 | 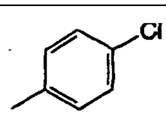 | 5-Cl | 3.12 |
| 90 | 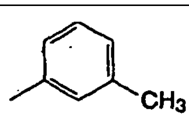 | 5-Cl | 3.03 |
| 91 | 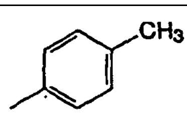 | 5-Cl | 3.03 |

(continued)

| No. | 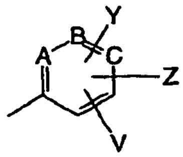 | X | k' |
|---|---|---|---|
| 92 | 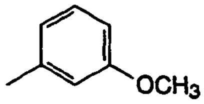 | 5-Cl | 2.94 |
| 93 | 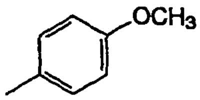 | 5-Cl | 2.95 |
| 94 | 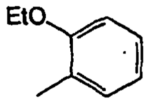 | 5-Cl | 3.17 |
| 95 | 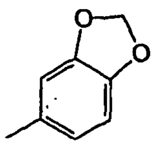 | 5-Cl | 2.9 |
| 96 | 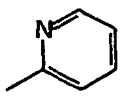 | 5-Cl | 1.94 |
| 97 | 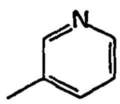 | 5-Cl | 1.39 |
| 98 | 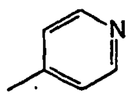 | 5-Cl | 1.23 |
| 99 | 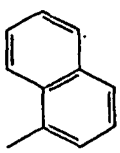 | 5-Cl | 4.23 |
| 100 | 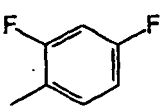 | 5-Cl | 3.88 |
| 101 | 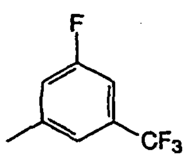 | 5-Cl | 4.055 |
| 102 | 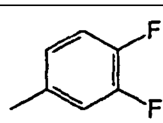 | 5-Cl | 3.91 |

(continued)

| No. | 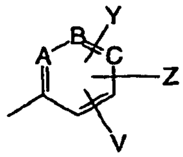 | X | k' |
|---|---|---|---|
| 103 | 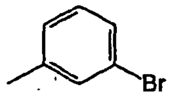 | 5-Cl | 4.07 |
| 104 | 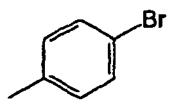 | 5-Cl | 4.01 |
| 105 | 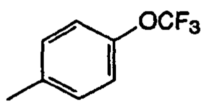 | 5-Cl | 4.23 |
| 106 | 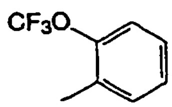 | 5-Cl | 3.762 |
| 107 | 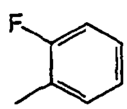 | 5-Cl | 3.86 |
| 108 | 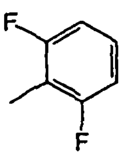 | 5-Cl | 3.66 |
| 109 | 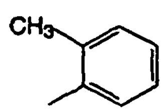 | 5-Cl | 3.89 |
| 110 | 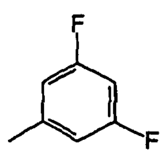 | 5-Cl | 3.86 |
| 111 | 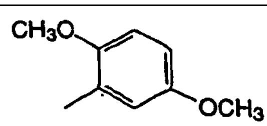 | 5-Cl | 3.85 |
| 112 | 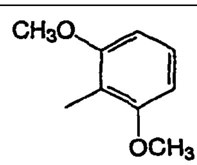 | 5-Cl | 3.9 |
| 113 | 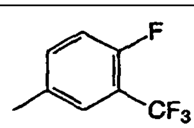 | 5-Cl | 4.13 |

(continued)

| No. | 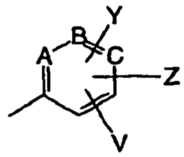 | X | k' |
|---|---|---|---|
| 114 | 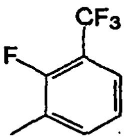 | 5-Cl | 4.04 |
| 115 | 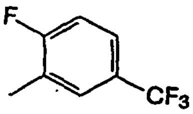 | 5-Cl | 4.08 |
| 116 | 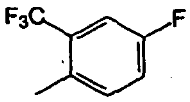 | 5-Cl | 3.97 |
| 117 | 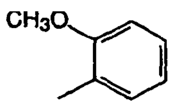 | 5-Cl | 6.55 |
| 118 | 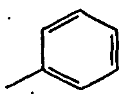 | 5-$CH_3O$ | 2.51 |
| 119 | 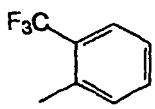 | 5-$CH_3O$ | 2.81 |
| 120 | 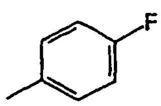 | 5-$CH_3O$ | 2.55 |
| 121 . | 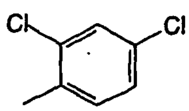 | 5-$CH_3O$ | 3.04 |
| 122 | 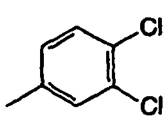 | 5-$CH_3O$ | 3.14 |
| 123 | 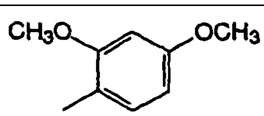 | 5-$CH_3O$ | 2.71 |
| 124 | 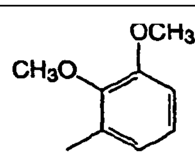 | 5-$CH_3O$ | 2.76 |

(continued)

| No. | 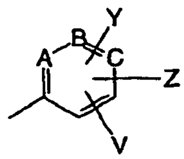 | X | k' |
|---|---|---|---|
| 125 | 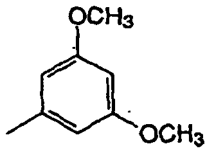 | 5-$CH_3O$ | 2.52 |
| 126 | 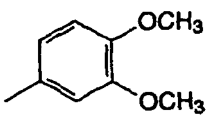 | 5-$CH_3O$ | 2.54 |
| 127 | 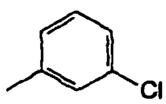 | 5-$CH_3O$ | 2.83 |
| 128 | 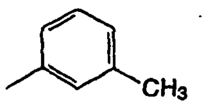 | 5-$CH_3O$ | 2.73 |
| 129 | 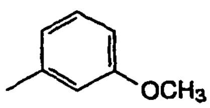 | 5-$CH_3O$ | 2.68 |
| 130 | 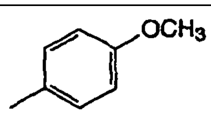 | 5-$CH_3O$ | 2.61 |
| 131 | 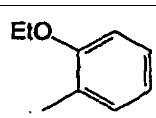 | 5-$CH_3O$ | 2.85. |
| 132 | 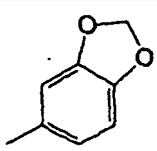 | 5-$CH_3O$ | 2.65 |
| 133 | 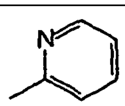 | 5-$CH_3O$ | 1.62 |
| 134 | 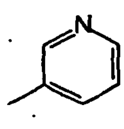 | 5-$CH_3O$ | 0.45 |
| 135 | 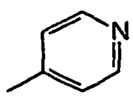 | 5-$CH_3O$ | 1.03 |

(continued)

| No. | 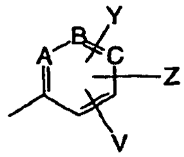 | X | k' |
|---|---|---|---|
| 136 | 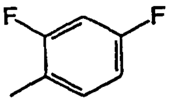 | 5-$CH_3O$ | 3.46 |
| 137 | 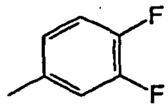 | 5-$CH_3O$ | 3.58 |
| 138 | 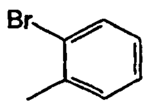 | 5-$CH_3O$ | 3.65 |
| 139 | 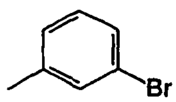 | 5-$CH_3O$ | 3.69 |
| 140 | 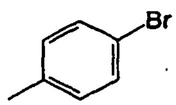 | 5-$CH_3O$ | 3.79 |
| 141 | 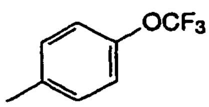 | 5-$CH_3O$ | 3.88 |
| 142 | 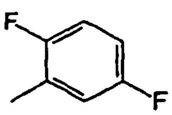 | 5-$CH_3O$ | 3.42 |
| 143 | 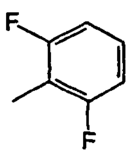 | 5-$CH_3O$ | 3.34 |
| 144 | 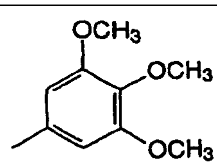 | 5-$CH_3O$ | 3.32 |
| 145 | 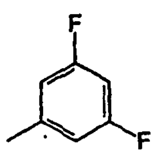 | 5-$CH_3O$ | 3.52. |
| 146 | 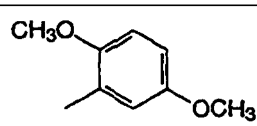 | 5-$CH_3O$ | 3.51 |

(continued)

| No. | 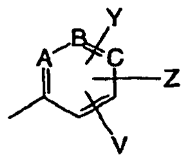 | X | k' |
|---|---|---|---|
| 147 | 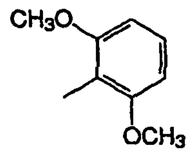 | 5-$CH_3O$ | 3.61 |
| 148 | 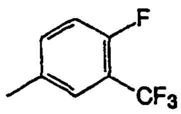 | 5-$CH_3O$ | 3.88 |
| 149 | 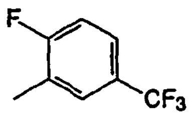 | 5-$CH_3O$ | 3.74 |
| 150 | 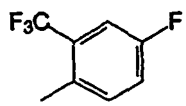 | 5-$CH_3O$ | 3.65 |
| 151 | 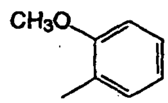 | 5-$CH_3O$ | 5.56 |

The used aralkylamine derivatives are commercial products.

**Example 2**

**Preparation of pharmaceutical compositions:**

**a) Tablets:**

0.01-50 % of active ingredient of formula I,15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin are mixed, then are granulated by wet granulation and pressed to tablets.

**b) Dragées, filmcoated tablets:**

The tablets made according to the method described above are coated by a layer consisting of entero- or gastrosolvent film, or of sugar and talc. The dragées are polished by a mixture of beeswax and carnuba wax.

**c) Capsules:**

0.01-50 % of active ingredient of formula I, 1-5 % of sodium lauryl sulfate, 15-50 % of starch, 15-50 % of lactose, 1-3 % of colloid silicon dioxide and 0.01-3 % of magnesium stearate are thoroughly mixed, the mixture is passed through a sieve and filled in hard gelatin capsules.

**d) Suspensions:**

Ingredients: 0.01-15 % of active ingredient of formula I, 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic

acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol is added in small portions under vigorous stirring, and the solution is left to stand for 10-12 h. Then the sodium hydroxide in 1 ml of distilled water, the aqueous solution of sorbitol and finally the ethanolic raspberry flavor are added with stirring. To this carrier the active ingredient is added in small portions and suspended with an immersing homogenizator. Finally the suspension is filled up to the desired final volume with distilled water and the suspension syrup is passed through a colloid milling equipment.

**e) Suppositories:**

For each suppository 0.01-15% of active ingredient of formula I and 1-20% of lactose are thoroughly mixed, then 50-95% of adeps pro suppository (for example Witepsol 4) is melted, cooled to 35 ˚C and the mixture of active ingredient and lactose is mixed in it with homogenizator. The obtained mixture is mould in cooled forms.

**f) Lyophilized powder ampoule compositions:**

A 5 % solution of mannitol or lactose is made with bidistilled water for injection use, and the solution is filtered so as to have sterile solution. A 0.01-5 % solution of the active ingredient of formula I is also made with bidistilled water for injection use, and this solution is filtered so as to have sterile solution. These two solutions are mixed under aseptic conditions, filled in 1 ml portions into ampoules, the content of the ampoules is lyophilized, and the ampoules are sealed under nitrogen. The contents of the ampoules are dissolved in sterile water or 0.9 % (physiological) sterile aqueous sodium chloride solution before administration.

## Claims

1. Indole-2-carboxamidine derivatives of formula (I)

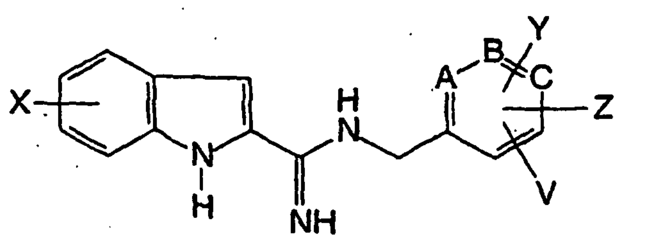

(I)

- wherein the meaning of

X is hydrogen or halogen atom, $C_1$-$C_4$ alkyl, $C_1$-$C_4$ alkoxy, trifluoromethyl group,
Y, V and Z independently are hydrogen or halogen atom, hydroxy, cyano, $C_1$-$C_4$ alkylsulfonamido optionally substituted by a halogen atom or halogen atoms, $C_1$-$C_4$ alkanoylamido optionally substituted by a halogen atom or halogen atoms, trifluoromethyl, trifluoromethoxy, $C_1$-$C_4$ alkyl, $C_1$-$C_4$ alkoxy group, or
the neighboring V and Z groups in given case together with one or more identical or different additional hetero atom and -CH= and/or -$CH_2$- groups can form a 4-7 membered homo- or heterocyclic ring, preferably benzene, dioxolane

A, B and C independently are -CH= group or one of them is nitrogen atom,
and the salts thereof:

2. A compound of the following group of indole-2-carboxamidine derivatives belonging to the scope of claim 1

N-(2,6-dimethoxy-benzyl)-1H-indole-2-carboxamidin,
N-(2-ethoxy-benzyl)-1H-indole-2-carboxamidine,
N-(3-methoxy-benzyl)-5-methoxy-1H-indole-2-carboxamidine,
N-(2,6-dimethoxy-benzyl)-5-chloro-1H-indole-2-carboxamidine,

N-(2,6-dimethoxy-benzyl)-5-methoxy-1H-indole-2-carboxamidine,
N-(2-ethoxy-benzyl)-5-fluoro-1H-indole-2-carboxamidine,
N-(2,6-dimethoxy-benzyl)-5-fluoro-1H-indole-2-carboxamidine
and the salts thereof.

3. Pharmaceutical compositions containing an effective amount of the indole-2-carboxamidine derivatives of formula (I) - wherein the meaning of A, B, C, X, Y, V, Z are as given in claim 1 - or the salts thereof as active ingredient and auxiliary materials, which are commonly used in practice, such as carriers, excipients, diluents, stabilizers, wetting or emulsifying agents, pH- and osmotic pressure-influencing, flavoring or aromatizing, as well as formulation-promoting or formulation-providing additives.

4. Process for preparing the indole-2-carboxamidine derivatives of formula (I), - wherein the meaning of A, B, C, X, Y, V, Z are as given in claim 1-, **characterized by**
reacting a carboximidic acid alkyl ester salt of formula (II)

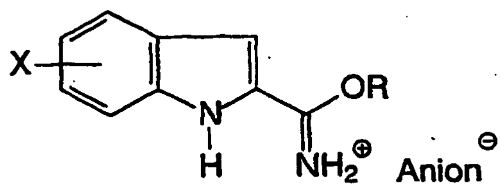

(II)

- wherein the meaning of X is as given in claim 1, Anion means one equivalent of an anion, R is $C_1$-$C_4$ alkyl - with an aralkylamine of formula (III)

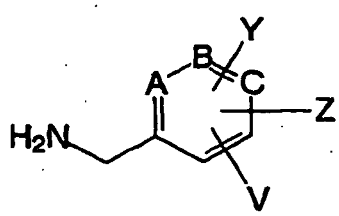

(III)

- wherein the meaning of A, B, C, Y, V, Z are as given in claim 1 -,

then transforming optionally the so obtained indole-2-carboxamidine derivatives of formula (I) - wherein the meaning of A, B, C, X, Y, V, Z are as given in claim 1 - into another compounds of formula (I) by introducing new substituents and/or modifying or removing the existing ones, and/or by forming salt and/or liberating the compound of formula (I) from salts by known methods.

5. Process as claimed in claim 4, **chacterized by** reacting the carboximidic acid alkyl ester hydrochloride of formula (II) - wherein the meaning of X and R is as given in claim 1, and Anion means $Cl^-$ ion - with the aralkylamine of formula (III) - wherein the meaning of A, B, C, Y, V, Z are as given in claim 1- in the presence of a base.

6. Process as claimed in claim 4, **chacterized by** reacting the carboximidic acid alkyl ester hydrochloride of formula (II) - wherein the meaning of X and R is as given in claim 1, and Anion means $Cl^-$ ion - with the aralkylamine of formula (III) - wherein the meaning of A, B, C, Y, V, Z are as given in claim 1- in the presence of excess of the aralkylamine of formula (III) - wherein the meaning of A, B, C, Y, V, Z are as given in claim 1-, in an alkanol.

7. Process for manufacturing pharmaceutical compositions having NR2B selective NMDA receptor antagonist effect, **characterized by** mixing a indole-2-carboxamidine derivative of formula (I) - wherein the meaning of A, B, C, X, Y, V, Z are as given in claim 1 - or the pharmaceutically acceptable salts thereof as active ingredient and auxiliary materials, which are commonly used in practice, such as carriers, excipients, diluents, stabilizers, wetting or emulsifying agents, pH- and osmotic pressure-influencing, flavoring or aromatizing, as well as formulation-promoting or formulation-providing additives.

**8.** Use of an indole-2-carboxamidine derivative of formula (I) - wherein the meaning of A, B, C, X, Y, V, Z are as given in claim 1 - or optical antipodes or racemates or the pharmaceutically acceptable salts thereof for the preparation of a pharmaceutical for the treatment and alleviation of symptoms of the following diseases in a mammal, including humans: traumatic injury of brain or spinal cord, human immunodeficiency virus (HIV) related neuronal injury, amyotrophic lateral sclerosis, tolerance and/or dependence to opioid treatment of pain, withdrawal syndromes of e.g. alcohol, opioids or cocaine, ischemic CNS disorders, chronic neurodegenerative disorders, such as Alzheimer's disease, Parkinson's disease, Huntington's disease, pain and chronic pain states, such as neuropathic pain or cancer related pain, epilepsy, anxiety, depression, migraine, psychosis, muscular spasm, dementia of various origin, hypoglycemia, degenerative disorders of the retina, glaucoma, asthma, tinnitus, aminoglycoside antibiotic-induced hearing loss.

## Patentansprüche

**1.** Indol-2-carboxamidinderivate der Formel (I):

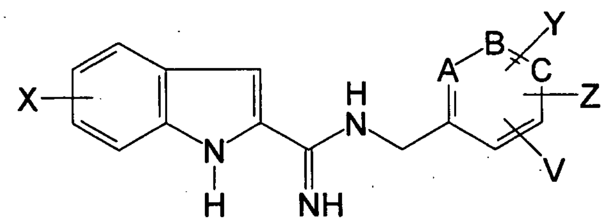

(I)

worin:

X ein Wasserstoff- oder Halogenatom, $C_{1-4}$-Alkyl, $C_{1-4}$-Alkoxy oder Trifluormethylgruppe bedeutet,
Y, V und Z unabhängig voneinander ein Wasserstoff- oder Halogenatom, Hydroxy, Cyano, $C_{1-4}$-Alkylsulfonamido, das gegebenenfalls mit einem Halogenatom oder Halogenatomen substituiert ist, $C_{1-4}$-Alkanoylamido, das gegebenenfalls mit einem Halogenatom oder Halogenatomen substituiert ist, Trifluormethyl, Trifluormethoxy, $C_{1-4}$-Alkyl oder eine $C_{1-4}$-Alkoxygruppe sind, oder
benachbarte V- und Z-Gruppen gegebenenfalls mit einem oder mehreren, identischen oder verschiedenen zusätzlichen Heteroatomen und (-CH=)- und/oder ($-CH_2$)-Gruppen einen 4- bis 7-gliedrigen homo- oder heterocyclischen Ring bilden können, vorzugsweise einen Benzol- oder Dioxolanring,
A, B und C unabhängig voneinander (-CH=)-Gruppen sind oder eines von diesen ein Stickstoffatom ist,
und Salze davon.

**2.** Verbindung aus der folgenden Gruppe von Indol-2-carboxamidinderivaten, die unter den Bereich von Anspruch 1 fallen:

N-(2,6-Dimethoxy-benzyl)-1H-indol-2-carboxamidin,
N-(2-Ethoxy-benzyl)-1H-indol-2-carboxamidin,
N-(3-Methoxy-benzyl)-5-methoxy-1H-indol-2-carboxamidin,
N-(2,6-Dimethoxy-benzyl)-5-chlor-1H-indol-2-carboxamidin,
N-(2,6-Dimethoxy-benzyl)-5-methoxy-1H-indol-2-carboxamidin,
N-(2-Ethoxy-benzyl)-5-fluor-1H-indol-2-carboxamidin,
N-(2,6-Dimethoxy-benzyl)-5-fluor-1H-indol-2-carboxamidin

und Salze davon.

**3.** Pharmazeutische Zusammensetzungen, die eine wirksame Menge der Indol-2-carboxamidinderivate der Formel (I), worin die Bedeutungen von A, B, C, X, Y, V und Z wie in Anspruch 1 angegeben sind, oder Salze davon als Wirkstoff enthalten, sowie üblicherweise in der Praxis verwendete Hilfsmaterialien, wie beispielsweise Träger, Hilfsstoffe, Verdünnungsmittel, Stabilisatoren, Feuchthalte- oder emulgierende Mittel, den pH-Wert oder osmotischen Druck beeinflussende, geschmackstragende oder aromatisierende sowie formulierungsfördernde oder formulierungsbildende Additive.

4. Verfahren zur Herstellung der Indol-2-carboxamidinderivate der Formel (I), worin die Bedeutungen von A, B, C, X, Y, V und Z so wie in Anspruch 1 angegeben sind, **gekennzeichnet durch** Umsetzen eines Carboximidsäurealkylestersalzes der Formel (II) :

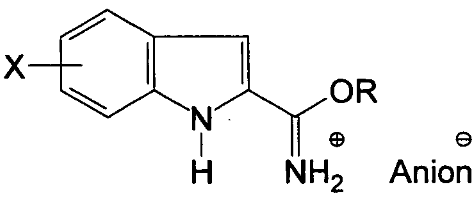

( II )

worin die Bedeutung von X wie in Anspruch 1 angegeben ist, "Anion" für ein Äquivalent eines Anions steht und R $C_{1-4}$-Alkyl ist, mit einem Aralkylamin der Formel (III)

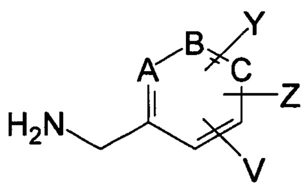

( III )

worin die Bedeutung von A, B, C, Y, V und Z wie in Anspruch 1 angegeben ist, anschliessend gegebenenfalls Umwandeln der so erhaltenen Indol-2-carboxamidinderivate der Formel (I), worin die Bedeutungen von A, B, C, X, Y, V und Z wie in Anspruch 1 angegeben sind, in andere Verbindungen der Formel (I) **durch** Einführen neuer Substituenten und/oder Modifizieren oder Entfernen der bestehenden Substituenten und/oder **durch** Salzbildung und/oder Freisetzen der Verbindung der Formel (I) aus Salzen **durch** bekannte Verfahren.

5. Verfahren gemäss Anspruch 4, **gekennzeichnet durch** Umsetzen des Carboximidsäurealkylester-hydrochlorids der Formel (II), worin die Bedeutungen von X und R wie in Anspruch 1 angegeben sind und Anion für ein $Cl^-$-Ion steht, mit dem Aralkylamin der Formel (III), worin die Bedeutungen von A, B, C, Y, V und Z wie in Anspruch 1 angegeben sind, in Gegenwart einer Base.

6. Verfahren gemäss Anspruch 4, **gekennzeichnet durch** Umsetzen des Carboximidsäurealkylester-hydrochlorids der Formel (II), worin die Bedeutungen von X und R wie in Anspruch 1 angegeben sind und Anion für ein $Cl^-$-Ion steht, mit dem Aralkylamin der Formel (III), worin die Bedeutungen von A, B, C, Y, V und Z wie in Anspruch 1 angegeben sind, in Gegenwart eines Überschusses des Aralkylamins der Formel (III), worin die Bedeutungen von A, B, C, Y, V und Z wie in Anspruch 1 angegeben sind, in einem Alkanol.

7. Verfahren zur Herstellung pharmazeutischer Zusammensetzungen mit NR2B-selektiver NMDA-Rezeptorantagonistischer Wirkung, **gekennzeichnet durch** Mischen eines Indol-2-carboxamidinderivats der Formel (I) als Wirkstoff, worin die Bedeutungen von A, B, C, X, Y, V und Z wie in Anspruch 1 angegeben sind, oder dessen pharmazeutisch annehmbarer Salze, und Hilfsmaterialien, wie sie üblicherweise in der Praxis verwendet werden, wie Träger, Hilfsstoffe, Verdünnungsmittel, Stabilisatoren, befeuchtende oder emulgierende Mittel, den pH-Wert oder osmotischen Druck beeinflussende, geschmackstragende oder aromatisierende sowie formulierungsfördernde oder formulierungsbildende Additive.

8. Verwendung eines Indol-2-carboxamidinderivats der Formel (I), worin die Bedeutungen von A, B, C, X, Y, V und Z wie in Anspruch 1 angegeben sind, oder optischer Antipoden oder Racemate oder deren pharmazeutisch annehmbaren Salze zur Herstellung eines Pharmazeutikums zur Behandlung und zur Linderung der Symptome der folgenden Krankheiten in Säugern, einschliesslich Menschen: traumatische Verletzung des Gehirns oder Rückenmarks, neuronale Verletzungen in Verbindung mit dem humanen Immunschwächevirus (HIV), amyotrophe Lateralsklerose, Toleranz und/oder Abhängigkeit gegenüber opioider Schmerzbehandlung, Entzugssyndrome von beispielsweise Alkohol, Opioiden oder Kokain, ischämische ZNS-Störungen, chronische neurodegenerative Störungen, wie beispielsweise Alzheimer-Krankheit, Parkinson-Krankheit, Huntington-Krankheit, Schmerz- und chronische Schmerzzustände, wie beispielsweise neuropathischer Schmerz oder Schmerz in Verbindung mit Krebs, Epilepsie, Angstzustände, Depression, Migräne, Psychose, Muskelspasmus, Demenz verschiedenen Ursprungs, Hypoglykämie, degenerative Störungen der Netzhaut, Glaukom, Asthma, Tinnitus, durch Aminoglycosid-Antibiotika verursachter Gehörverlust.

**Revendications**

**1.** Dérivés d'indole-2-carboxamidine de formule (I)

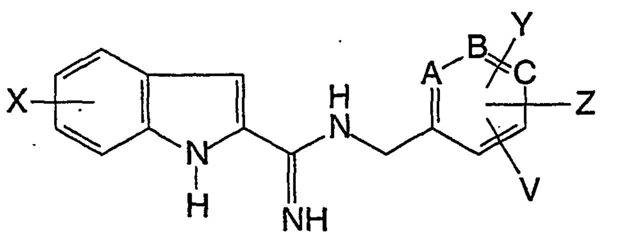

(I)

dans laquelle
X est un atome d'hydrogène ou un atome d'halogène, un groupe alkyle en $C_1$-$C_4$, un groupe alcoxy en $C_1$-$C_4$ ou un groupe trifluorométhyle,
Y, V et Z sont indépendamment un atome d'hydrogène ou un atome d'halogène, un groupe hydroxy, un groupe cyano, un groupe alkylsulfonamido en $C_1$-$C_4$ éventuellement substitué par un atome d'halogène ou des atomes d'halogène, un groupe alcanoylamido en $C_1$-$C_4$ éventuellement substitué par un atome d'halogène ou des atomes d'halogène, un groupe trifluorométhyle, un groupe trifluorométhoxy, un groupe alkyle en $C_1$-$C_4$, un groupe alcoxy en $C_1$-$C_4$, ou
les groupes V et Z voisins dans un cas donné, ensemble avec un ou plusieurs hétéroatomes supplémentaires identiques ou différents et les groupes -CH= et/ou -$CH_2$- forment un cycle homo- ou hétérocyclique de 4 à 7 éléments, de préférence, un cycle benzène ou un cycle dioxolane,
A, B et C sont indépendamment un groupe -CH= ou l'un d'eux est un atome d'azote,
et leurs sels.

**2.** Composé du groupe suivant de dérivés d'indole-2-carboxamidine selon la revendication 1

N-(2,6-diméthoxy-benzyl)-1H-indole-2-carboxamidine,
N-(2-éthoxy-benzyl)-1H-indole-2-carboxamidine,
N-(3-méthoxy-benzyl)-5-méthoxy-1H-indole-2-carboxamidine,
N-(2,6-diméthoxy-benzyl)-5-chloro-1H-indole-2-carboxamidine,
N-(2,6-diméthoxy-benzyl)-5-méthoxy-1H-indole-2-carboxamidine,
N-(2-éthoxy-benzyl)-5-fluoro-1H-indole-2-carboxamidine,
N-(2,6-diméthoxy-benzyl)-5-fluoro-1H-indole-2-carboxamidine,

et leurs sels.

**3.** Compositions pharmaceutiques contenant une quantité efficace des dérivés d'indole-2-carboxamidine de formule (I) où A, B, C, X, Y, V et Z sont tels que décrits dans la revendication 1, ou de leurs sels en tant qu'ingrédient actif et des composés auxiliaires qui sont habituellement utilisés dans la pratique tels que les supports, les excipients, les diluants, les agents stabilisants, les agents mouillants ou les agents émulsifiants, les agents influençant le pH et la pression osmotique, les agents aromatisants, ainsi que les additifs favorisant la formulation ou donnant la formulation.

**4.** Procédé pour la préparation des dérivés d'indole-2-carboxamidine de formule (I) où A, B, C, X, Y, V et Z sont tels que décrits dans la revendication 1, **caractérisé par**
la réaction d'un sel d'ester alkylique d'acide carboximidique de formule (II)

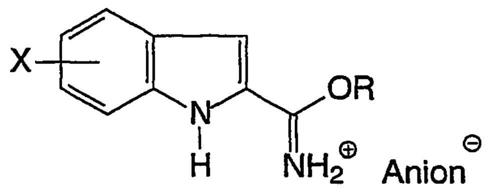

(II)

où X est tel que décrit dans la revendication 1, Anion signifie un équivalent d'un anion, R est un groupe alkyle en $C_1$-$C_4$, avec une aralkylamine de formule (III)

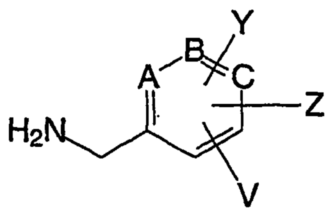

(III)

où A, B, C, Y, V et Z sont tels que décrits dans la revendication 1,
la transformation éventuelle ensuite des dérivés d'indole-2-carboxamidine de formule (I) ainsi obtenus où A, B, C, X, Y, V et Z sont tells que décrits dans la revendication 1, en d'autres composés de formule (I) en introduisant de nouveaux substituants et/ou en modifiant ou en éliminant ceux existants, et/ou en formant un sel et/ou en libérant le composé de formule (I) des sels par des procédés connus.

**5.** Procédé selon la revendication 4, **caractérisé par** la réaction du chlorhydrate d'ester alkylique d'acide carboximidique de formule (II) où X et R sont tels que décrits dans la revendication 1 et Anion signifie un ion $Cl^-$, avec l'aralkylamine de formule (III) où A, B, C, Y, V et Z sont tels que décrits dans la revendication 1, en présence d'une base.

**6.** Procédé selon la revendication 4, **caractérisé par** la réaction du chlorhydrate d'ester alkylique d'acide carboximidique de formule (II) où X et R sont tels que décrits dans la revendication 1 et Anion signifie un ion $Cl^-$, avec l'aralkylamine de formule (III) où A, B, C, Y, V et Z sont tels que décrits dans la revendication 1, en présence d'un excès d'aralkylamine de formule (III) où A, B, C, Y, V et Z sont tels que décrits dans la revendication 1, dans un alcanol.

**7.** Procédé pour la fabrication de compositions pharmaceutiques ayant un effet antagoniste sur le récepteur NMDA sélectif de NR2B, **caractérisé par** le mélange d'un dérivé d'indole-2-carboxamidine de formule (I) où A, B, C, X, Y, V et Z sont tels que décrits dans la revendication 1, ou de sels pharmaceutiquement acceptables de celui-ci en tant qu'ingrédient actif et de composés auxiliaires qui sont habituellement utilisés dans la pratique tels que les supports, les excipients, les diluants, les agents stabilisants, les agents mouillants ou les agents émulsifiants, les agents influençant le pH et la pression osmotique, les agents aromatisants, ainsi que les additifs favorisant la formulation ou donnant la formulation.

**8.** Utilisation d'un dérivé d'indole-2-carboxamidine de formule (I) où A, B, C, X, Y, V et Z sont tels que décrits dans la revendication 1, ou d'antipodes optiques ou de racémates ou de sels pharmaceutiquement acceptable de celui-ci pour la préparation d'un produit pharmaceutique destiné au traitement et au soulagement des symptômes des maladies suivantes chez les mammifères, incluant les êtres humains une lésion traumatique du cerveau ou de la moelle épinière, une lésion neuronale liée au virus de l'immunodéficience humaine (VIH), la sclérose latérale amyotrophique, la tolérance et/ou la dépendance à un traitement de la douleur à base d'un opioïde, les syndromes de sevrage à, par exemple l'alcool, un opioïde ou la cocaïne, les troubles ischémiques du SNC, les troubles neurodégénératifs chroniques tels que la maladie d'Alzheimer, la maladie de Parkinson, la maladie de Huntington, la douleur et les états de douleur chronique tels que la douleur neuropathique ou une douleur liée à un cancer, l'épilepsie, l'anxiété, la dépression, la migraine, la psychose, le spasme musculaire, la démence de diverses origines, l'hypoglycémie, les troubles dégénératifs de la rétine, le glaucome, l’asthme, les acouphènes et la perte auditive induite par un antibiotique aminoglycoside.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200198262 A
- WO 200067751 A
- WO 0000197 A


### Non-patent literature cited in the description


- *TINS,* 1987, vol. 10, 299-302
- *Stroke,* 1997, vol. 28, 2244-2251
- *Brain Res.,* 1998, vol. 792, 291-298
- *Exp. Neurol.,* 2000, vol. 163, 239-243
- *Neuropharmacology,* 1999, vol. 38, 611-623
- **C.N. SANG et al.** Program No. 814.9. 2003 Abstract Viewer/Itinerary Planner. *Society for Neuroscience,* 2003
- *J. Neurochem.,* 1998, vol. 70, 2147-2155
- *Bioorg. Med. Chem. Letters,* 2003, vol. 13, 693-696
- *Bioorg. Med. Chem. Letters,* 2003, vol. 13, 697-700
- *Biotechniques,* May 1997, vol. 22 (5), 982-7
- *Neurochemistry International,* 2003, vol. 43, 19-29
- Primary cell cultures of peripheral and central neurons and glia. **JOHNSON, M.I. ; BUNGE, R.P.** Protocols for Neural Cell Culture. The Humana Press Inc, 1992, 51-75
- *Neurol. Res.,* 1999, vol. 21, 309-12
- *Drug and Alcohol Depend.,* 2000, vol. 59, 1-15
- *Neurosci. Lett.,* 1987, vol. 73, 143-148
- *Expert Opin. Investig. Drugs,* 2000, vol. 9, 1397-406
- *Drug News Perspect,* 1998, vol. 11, 523-569